# EUROPEAN PATENT APPLICATION

(11) **EP 4 459 627 A1**
(43) Date of publication of application: **06.11.2024**
(21) Application number: 24172940.9
(22) Date of filing: 29.04.2024
(51) Int. Cl.: G16H 10/40

(54) **METHOD AND SYSTEM**

(30) Priority: 05.05.2023 EP 23171961
(71) Applicant: F. Hoffmann-La Roche AG, 4070 Basel (CH); Roche Diagnostics GmbH, 68305 Mannheim (DE)
(72) Inventor: GLAUSER, Michael, 6343 Rotkreuz ZG (CH); GUTMANN, Oliver, 6343 Rotkreuz ZG (CH); JANNER, Gabriele Piero, 6343 Rotkreuz ZG (CH); SAROFIM, Emad, 6343 Rotkreuz ZG (CH); THOMANN, Marcel, 6343 Rotkreuz ZG (CH); VOLLENWEIDER, Urs, 6343 Rotkreuz ZG (CH); SCHWEIGHAUSER, Stephan, 8153 Rümlang (CH)
(74) Representative: Herren, Barbara

(57) **Abstract**

A computer-implemented method for optimising an assignment of one or more medical samples to one or more analytical tests to be conducted on those medical samples. The method comprises the steps of: obtaining an initial assignment (100) of the one or more medical samples to the one or more analytical tests; determining a compliance status for the or each medical sample, the compliance status indicating: (i) whether a sample quality metric of the or each medical sample violates an analytical test specification (300) of the medical sample's assigned analytical test(s) in the initial assignment (100) and/or (ii) whether the or each medical sample is unprocessable; and performing a mitigation action if one or more compliance status indicate: (i) that the sample quality metric of a given medical sample violates the analytical test specification (300) and/or (ii) a given medical sample is unprocessable.

## Description

### Field of the Invention

The present invention relates to a computer-implemented method for optimising the assignment of a medical sample to an analytical test and an analytical testing management system.

### Background

According to the literature, roughly two-thirds of the errors and invalid results related to laboratory testing originate in the collection, handling and processing of the sample before it arrives at the laboratory.

Prior to the arrival of a sample at a laboratory, there are several activities undertaken that have the ability to influence the success of subsequent laboratory testing, starting from the collection of patient samples. Multiple factors can affect the quality of the samples (e.g. time, temperature exposure, light exposure, shock, humidity).

Laboratory testing that results in errors and invalid results is undesirable for several reasons. Firstly, it wastes the time of patients, medical professionals and laboratory staff. Additionally, it may delay subsequent medical intervention based on the results of those lab tests, which may be detrimental to patient outcomes. It is also a waste of physical resources, both in the collection of samples and the testing of those samples. Finally, carrying out testing that results in invalid results reduces useful lab throughput (i.e. the throughput of samples that result in valid test results).

The present invention has been devised in light of the above considerations.

### Summary of the Invention

According to a first aspect there is provided a computer-implemented method for optimising an assignment of one or more medical samples to one or more analytical tests to be conducted on those medical samples prior to processing of said medical samples in an analytical laboratory. The method of the first aspect comprises the steps of: obtaining an initial assignment of the one or more medical samples to the one or more analytical tests; determining a compliance status for the or each medical sample, the compliance status indicating: (i) whether a sample quality metric of the or each medical sample violates an analytical test specification of the medical sample's assigned analytical test(s) in the initial assignment and/or (ii) whether the or each medical sample is unprocessable; and performing, prior to processing of said medical sample, a mitigation action if one or more compliance status indicate: (i) that the sample quality metric of a given medical sample violates the analytical test specification and/or (ii) a given medical sample is unprocessable, wherein the mitigation action includes a step of modifying the assignment of the medical sample(s) to the analytical test(s) based on the or each compliance status.

Such a method is able to determine whether a given medical sample is compliant with an analytical test it is assigned to, and therefore whether the assigned analytical test (i) is likely to provide a valid result if conducted on the medical sample and/or (ii) can be conducted on the medical sample (irrespective of whether the result is likely to be valid or invalid), respectively. By taking a mitigation action where the result of this determination requires, the method reduces wasteful consumption of resources (e.g. both lab resources and sample collection resources) and can increase useful lab throughput. Additionally, better patient health outcomes may be obtained where the above method allows patients to avoid waiting for analytical test results that prove to be invalid due to poor sample quality, and therefore receive suitable medical intervention earlier.

The above-described method may be executed by an analytical testing management system configured to communicate with a Laboratory Scheduling System and possibly with one or more remote agents.

It is understood that an assignment of one or more medical samples to one or more analytical tests to be conducted on those medical samples is optimized prior to processing of said medical samples in an analytical laboratory. Processing may be either performing a sample quality test or performing a test according to an analytical test specification on the sample itself. For example, an aliquot of the sample may be analysed for a specific quality relevant analyte to determine the quality of the sample. Or one or more analytical tests may be conducted on the medical sample or an aliquot thereof. By performing the present computer-implemented method of optimising an assignment of one or more medical samples to one or more analytical tests to be conducted on those medical samples prior to processing of said medical samples in an analytical laboratory, a waste of lab resources and/or reagents can be avoided and useful lab throughput increased. The method may be performed by an analytical testing management system which is communicatively connected to a Laboratory Scheduling System (20). The analytical testing management system obtains the initial assignment from the Laboratory Scheduling System (20) prior to processing of the medical sample according to the initial assignment.

A medical sample that is stated to be "compliant" with an analytical test may be understood to (i) have a sample quality metric that complies with (i.e. does not violate) the analytical test specification and (ii) is processable (i.e. is not unprocessable).

The analytical test(s) may be ones performed on or suitable for performance on an in-vitro diagnostic instrument. They may determine, for example, one or more clinically relevant values from a sample (e.g. blood glucose level) and/or an indication of one or more clinically relevant markers (e.g. presence of virus, or enzyme). The in-vitro diagnostic instrument may be a laboratory instrument or a point-of-care device.

The determination of the compliance status may include only determining whether a sample quality metric of the or each medical sample violates an analytical test specification of the medical sample's assigned analytical test(s) in the initial assignment. The determination of the compliance status may include only determining whether the or each medical sample is unprocessable. In some examples, the determination of the compliance status may include both determining whether a sample quality metric of the or each medical sample violates an analytical test specification of the medical sample's assigned analytical test(s) in the initial assignment and determining whether the or each medical sample is unprocessable. In yet further examples, the determination of the compliance status may first include determining whether the or each medical sample is unprocessable and then determining whether a sample quality metric of the or each medical sample violates an analytical test specification of the medical sample's assigned analytical test(s) in the initial assignment only for medical samples which have been determined to be processable.

The mitigation action may include a step of modifying (i.e. automatically, by the computer) the initial assignment of the medical sample(s) to the analytical test(s) based on the or each compliance status in respect of the assigned analytical tests. By the mitigation action including a step of modifying the assignment of the medical sample(s) to the analytical test(s), it may be possible to reduce the number of medical sample(s) that are discarded because they are found not to comply with the analytical test specification of the test that were initially assigned to.

The mitigation action may include flagging and/or invalidating a test order associated with the medical sample and respective analytical test(s) based on the or each compliance status. Accordingly, it is possible to bring the invalid test order to the attention of a laboratory operator and prevent the test order from being performed or the result from being relied on.

A laboratory operator may resolve a flagged test order by, for example, performing the test using a Point of Care device, contacting the test order placer, or manually routing the test order into a laboratory schedule.

Where there are plural compliance status that each indicate that a sample quality metric violates a corresponding analytical test specification and/or a medical sample is unprocessable, it is possible for different mitigation actions to be taken in response to different negative compliance status. In some examples, where there are plural compliance status that each indicate that a sample quality metric violates a corresponding analytical test and/or a medical sample is unprocessable, the same mitigation action may be taken in response to the negative compliance status.

The mitigation action may include a step of notifying an operator of the violation of the analytical test specification. This notification may include: (i) one or more proposed modifications of the assignment of the medical sample(s) to analytical test(s); (ii) a notification or flag based on the or each compliance status. The mitigation action may further comprise that the operator can accept or decline the proposed modifications. The ability for the lab operator to decide to decline the proposed modifications and execute the test despite a sample quality metric violating an analytical test specification may be useful where it is desired to gain experience, to obtain an indicative result, to use the result for stability studies, or may be useful where there are time lag or other issues associated with obtaining an additional sample, or where the medical sample is expensive.

Where the compliance status indicates whether a medical sample is unprocessable, the step of determining a compliance status may further include determining whether the or each medical sample arrived at the intended destination (e.g. laboratory), was lost, was spilled or otherwise contaminated or tampered with, or if an identification label of the sample (e.g. barcode) was lost or rendered unreadable, or if the medical sample was not collected, for example, because there was insufficient time to collect the sample, because a patient's condition/state meant the sample could not be collected (e.g. blood not flowing well, veins of the patient not accessible), because a patient was not available to provide the sample, because of an error on behalf of the sample collector, because no more sample containers of the needed type were available, etc. This may assist in reducing the number of medical samples that have a compliance status indicating they are unprocessable by identifying the cause of the sample becoming unprocessable.

Where reference is made to a sample quality metric, said metric may comprise one or more parameters or statistics. The quality metric (whether a parameter or statistic) may have a threshold value above which the compliance status is held to be valid and below which the compliance status is held to be negative.

The method may further comprise a step of deriving the sample quality metric for the or each medical sample. Said step may include: obtaining sample data including one or more of: collection information for the sample, transport information from the sample, centrifugation information for the sample; and storage condition information of the sample; and deriving the sample quality metric based on the sample data. The sample data (200) may be obtained from a remote agent. The remote agent may be, e.g. one or more sensors in a transport device in which the samples are transported. The analytical testing management system may communicatively connect to said sensors and obtain the sample data from said sensors. In another embodiment, the remote agent may be a processor in a computer, e.g. located at the site of collection of the sample. Said processor may comprise sample data relating to the sample collection, e.g. on the patient's condition, or if the sample could not be appropriately collected or collection time etc. Further examples of such sample data is provided below. The analytical testing management system may communicatively connect to said processor to obtain said sample data (200).

The sensors may measure e.g. temperature or humidity etc. during transport.

Advantageously, the sample quality metric being based on said sample data means that the sample quality metric reflects the information that will be pertinent to whether the medical analytical test can be successfully conducted on that medical sample.

The collection information for a given sample may include one or more of: a collection time stamp, patient data, collection observations, images of the sample after collection and a sample fixation time.

The patient data may include one or more of: a patient ID, and patient demographics.

The transport information for the sample may include one or more of: a sample temperature measurement during transport, a sample light exposure measurement during transport, a sample shock exposure measurement during transport, sample container additive information, and GPS data.

The centrifugation information for the sample may include one or more of: an indication of whether centrifugation has been conducted, a collection-to-centrifugation time measurement and a centrifugation specification.

The storage condition information for the sample may include one or more of: a temperature measurement during storage, a sample light exposure measurement during storage, a humidity measurement during storage, a sample shock exposure measurement during storage, and a storage duration measurement.

The step of determining the compliance status of the or each medical sample in respect of the assigned analytical test may include either: obtaining, for the or each medical sample, from a remote device, an indication of whether the sample quality metric of the medical sample violates the analytical test specification of its assigned analytical test(s) in the initial assignment; or obtaining the sample quality metric for the or each medical sample; obtaining the analytical test specification for the or each analytical test in the initial assignment; and performing a compliance process of assessing, for the or each medical sample, whether the sample quality metric violates the analytical test specification of its assigned analytical test(s) in the initial assignment. Advantageously, determining the compliance status via a remote device can allow duplication of a compliance process to be avoided where the compliance process has already been conducted by the remote device. The determination of the compliance status through the computer system performing the compliance process (rather than obtaining the compliance status from a third party performing the compliance process) may be advantageous because the test specifications then do not need to be provided to a third party. In particular, where the test specifications are based, at least in part, on information obtained from a laboratory operator, not providing test specifications based on this data to third parties may be desirable from the perspective of information security and confidentiality.

The process of modifying the assignment may be conducted via a remote device or directly by the computer implementing the method. The modification may comprise a re-matching of the one or more medical samples to one or more analytical tests in the initial assignment. Such a re-matching may be based on the knowledge of which of the one or more analytical tests within the initial assignment each of the medical samples is compliant with. Advantageously, such a re-matching may allow all of the analytical tests present in the initial assignment to be validly conducted, even where the initial assignment of medical to samples to analytical tests would have resulted in one or more invalid tests being conducted.

The sample quality metric may contain haemoglobin, icterus and lipemia indices. In one embodiment, the sample quality metric does not include hemolysis indices (e.g. H-index).

The analytical test specification may be obtained from one or more of: IVD vendor information, lab requirements (via a lab operator), data indicative of best practices and general guidelines and tube manufacturer guidelines. Furthermore, analytical test specifications can be specific to an analytical test or to a family of tests arising from harmonized/standardized test codes, for example, from LOINC, SNOMED (CT), JLAC10 and/or C-NPU codes.

The analytical test specification may be obtained based on a harmonised/standardised test code and/or another description of the analytical test. Using both a test code and another description of the analytical test is advantageous where there is not a one-to-one mapping of test codes to analytical tests.

An analytical test specification for an analytical test may be obtained from a test supplier or a sample container manufacturer. Alternatively, the analytical test specification may be obtained from a database, the test specification in the database being based on one or more of: information obtained from a test supplier, information obtained from a sample container manufacturer, and information obtained from a laboratory operator. The database may be internal to the computer system or may be accessible by the computer system via a networked connection.

The method may further comprise a step of determining a sum of test specification violations with the initial assignment. Modifying the assignment may then include performing a minimisation optimisation with the sum of test specification violations as an objective function. Such a method can maximise the number of analytical tests listed within the initial assignment that can still be performed with a modified assignment of samples to analytical tests in a case where it is not possible to arrive at a modified assignment having no test specification violations.

The minimisation optimisation may be a method or algorithm which functions to minimise the total number, sum, or weighted sum of test specification violations by modifying the initial assignments based on a set of constraints and the relationship between one or more decision variables. The optimisation may use a model containing: one or more decision variables (for example, which test should be carried out on which sample), an objective function (for example, the total number, sum, or weighted sum of test specification violations), and one or more constraints (for example, not exceeding the available net volume of the samples). The data for the decision variables, objective function and constraints for an optimisation for a laboratory may be obtained from order data, test specifications and sample quality metrics of samples. The minimisation optimisation may be configured such that a priority ranking of the analytical tests to be performed is incorporated into the objective function alongside the number of test specification violations, for example, the weighted sum of test specification violations may use said priority ranking as the weights. By objective function, it may be meant any mathematical function which an optimisation attempts to maximise or minimise whilst complying with a set of constraints and accounting for the relationships between one or more decision variables.

The step of modifying the assignment may further comprise: identifying a medical sample whose sample quality metric violates the analytical test specification of its assigned analytical test(s) in the initial assignment; identifying, for that medical sample, an alternative analytical test other than its assigned analytical test(s) in the initial assignment that its sample quality metric does not violate the analytical test specification of; and modifying the assignment such that said medical sample is assigned to the alternative analytical test. Advantageously, this allows the method to make more efficient use of medical samples by avoiding a sample (and lab resources) being wasted by having a test that it is not compliant with the specification of performed on it, and by avoiding a sample being discarded because it is not compliant with the test initially assigned to it. These additional processes within the step of modifying the assignment may be conducted by a third party and provided to the analytical testing management system, or the analytical testing management system may itself conduct these additional processes.

The alternative analytical test may be a test present in the initial assignment. This is advantageous where the medical sample originally assigned to this alternative analytical test has a sample quality metric that will not violate the analytical test specification of the analytical test whose test specification was violated by the sample quality metric of the medical sample initially assigned to it. In such a case, a direct swap of the analytical tests assigned to the medical samples can be conducted.

Alternatively, the alternative analytical test may be a test that is not present in the initial assignment. The alternative analytical test may be a test that can be conducted for the same purpose as the assigned test in the initial assignment whose specification was violated by the sample metric of the initially assigned provider. For example, the alternative analytical test may be a test for the same purpose (and possibly even the same parameter) from a different provider. Advantageously, this may allow values for the same parameters to be obtained from the testing of the samples even where there is a test specification violation in the initial assignment of medical samples to analytical tests.

The method may further comprise a step of assessing, for the or each medical sample, whether its sample quality metric violates the analytical test specification of the or each of the analytical test(s) in the initial assignment other than its assigned analytical test(s) in the initial assignment. Such a step may be beneficial when subsequently executing the step of modifying the assignment of medical samples to analytical tests.

The step of modifying the assignment of medical samples to analytical tests may further comprise identifying a medical sample whose sample quality metric violates all of the analytical test specifications of the analytical tests in the initial assignment; and determining, for said medical sample, that there is not an alternative analytical test that is not present in the initial assignment that its sample quality metric does not violate the analytical test specification of; and removing said medical sample from the assignment. In one embodiment, the analytical test management system sends instructions to the Laboratory Scheduling System to remove said medical sample from said initial assignment before processing said medical sample. Advantageously, this avoids an analytical test being conducted on said medical sample where it has been established that the result of such an analytical test will be invalid, thereby reducing resource usage and facilitating an increase in useful laboratory throughput.

Where a medical sample has been removed from the assignment, the method may further comprise a step of requesting a new medical sample to replace said medical sample in the assignment. This facilitates the intended analytical test being performed to obtain the test result of interest once a new medical sample is provided. The requesting of a new sample can be done prior to processing the medical sample. This saves costs, resources and time.

The initial assignment may further comprise a priority ranking of the analytical tests, indicating the priority with which each test should be performed. The step of modifying the assignment may then be based in part on the priority ranking. Advantageously, this can ensure that the most important analytical tests are prioritised when modifying the assignment, such that they are still performed when not all of the analytical tests in the initial assignment can be performed even following the modification of the assignment.

The priority ranking may be a weighted sum, e.g. a weighted sum based on factors including: how suitable a given sample is for a given test (e.g. how comparable the results are clinically); whether the test is given an `URGENT' status or not; what fraction of a sample's volume is used by a given test; the time-sensitivity of obtaining a test result; and whether the test result is essential or supporting, for example, for a diagnosis.. The priority ranking may be based on a test order placer preference. The priority ranking may be based on a lab preference. Lab preference may reflect a time to result output, the stability of a sample to conduct the test on, sample container information (e.g. size, compatibility with instruments in the lab), the availability of reagents, and the status of instruments for the test.

The method may further comprise a step of distributing the modified assignment to a laboratory information system, healthcare information system or laboratory information management system.

The step of modifying the assignment may further comprise assigning a medical sample to more than one analytical test. Where a medical sample is assigned to plural analytical tests, the method may further comprise a step of instructing the creation of an aliquot from said sample. This allows a greater proportion of the analytical tests to be performed in the case where there is a one-to-one matching of medical samples to analytical tests and there would otherwise not be a compliant medical sample to assign to that analytical test.

The method may further comprise, following the modification of the assignment, forwarding instructions to a laboratory scheduling system to process the medical sample according to the modified assignment to generate a test result.

Thus, the benefits of the modified assignment, including avoiding delays to subsequent medical intervention caused by invalid test results, avoiding wasting resources conducting tests that provide invalid results and increasing lab throughput, can be realised without human intervention.

Alternatively, the modified assignment may be provided to a display means. In such a case, the method may further comprise providing an indication of the medical sample(s) that violated the analytical test specification(s) of their assigned analytical test(s) in the modified assignment.

In a second aspect there is provided an analytical testing management system for optimising an assignment of one or more medical samples to one or more analytical tests to be conducted on those medical samples. The system comprises an optimisation module configured to obtain an initial assignment of the one or more medical samples to the one or more analytical tests; and a compliance module configured to: determine, for the or each medical sample, a compliance status indicating: (i) whether a sample quality metric of the or each medical sample violates an analytical test specification of the medical sample's assigned analytical test(s) in the initial assignment and/or (ii) whether the or each medical sample is unprocessable. The optimisation module is further configured to perform a mitigation action prior to processing of said medical samples in an analytical laboratory if one or more compliance status indicates: (i) that the sample quality metric of a given medical sample violates the analytical test specification and/or (ii) whether a given sample is unprocessable, wherein the mitigation action includes a step of modifying the assignment of the medical sample(s) to the analytical test(s) based on the or each compliance status.

Alternatively, the optimisation module may be provided within a third-party system configured to communicate with the analytical testing management system.

Such a system is able to determine whether a given medical sample is compliant with an analytical test it is assigned to, and therefore whether the assigned analytical test (i) is likely to provide a valid result if conducted on the medical sample and/or (ii) can be conducted on the medical sample (irrespective of whether the result is likely to be valid or invalid). By taking a mitigation action where the result of this determination requires, the system reduces wasteful consumption of resources (e.g. both lab resources and sample collection resources) and can increase useful lab throughput. Additionally, better patient health outcomes may be obtained where the above method allows patients to avoid waiting for analytical test results that prove to be invalid due to poor sample quality, and therefore receive suitable medical intervention earlier.

The mitigation action may include modifying the assignment of the medical sample(s) to the analytical test(s) based on the or each compliance status in respect of the assigned analytical for the sample according to the modified assignment.. By the mitigation action including a step of modifying the assignment of the medical sample(s) to the analytical test(s), it may be possible to reduce the number of medical sample(s) that are discarded because they are found not to comply with the analytical test specification of the test that were originally assigned to.

The mitigation action may include a further step of flagging and/or invalidating a test order associated with the medical sample and respective analytical test(s) based on the or each compliance status. In a further embodiment, the flagging and/or invalidating of the test order results in a flag of the test result when the medical sample is processed according to the modified assignment.

Accordingly, it is possible to bring the invalid test order/result to the attention of a laboratory operator and prevent the test order/result from being relied on.

The mitigation action may include a step of notifying an operator of the violation of the analytical test specification. This notification may include (i) one or more proposed modifications of the assignment of the medical sample(s) to analytical test(s) (ii) a notification and/or flag based on the or each compliance status; and/or (iii) an indication that a test order and/or test result associated with the analytical test is invalid based on the or each compliance status.

The optimisation module may be configured to derive the sample quality metric for the or each medical sample by being configured to: obtain sample data including one or more of: collection information for the sample, transport information for the sample, centrifugation information for the sample; and storage condition information for the sample; and derive the sample quality metric based on the sample data.

The compliance module may be configured to determine the compliance status for the or each medical sample by being configured to either: obtain, for the or each medical sample, from a remote device, an indication of whether the sample quality metric of the medical sample violates the analytical test specification of its assigned analytical test(s) in the initial assignment; or: obtain the sample quality metric for the or each medical sample; obtain the analytical test specification for the or each analytical test in the initial assignment; and perform a compliance process of assessing, for the or each medical sample, whether the sample quality metric violates the analytical test specification (300) of its assigned analytical test(s) in the initial assignment.

The optimisation module may be configured to modify the assignment by being configured to: identify a medical sample whose sample quality metric violates the analytical test specification of its assigned analytical test(s) in the initial assignment; identify, for that medical sample, an alternative analytical test other than its assigned analytical test(s) in the initial assignment that its sample quality metric does not violate the analytical test specification of; and modify the assignment such that said medical sample is assigned to the alternative analytical test. The alternative analytical test may be a test present in the initial assignment or it may be a test not present in the initial assignment.

The compliance module may be further configured to assess, for the or each medical sample, whether its sample quality metric violates the analytical test specification of the or each of the analytical test(s) in the initial assignment other than its assigned analytical test(s) in the initial assignment.

The optimisation module may be configured to modify the assignment by being configured to: identify a medical sample whose sample quality metric violates all of the analytical test specifications of the analytical tests in the initial assignment; determine, for said medical sample, that there is not an alternative analytical test that is not present in the initial assignment that its sample quality metric does not violate the analytical test specification of; and remove said medical sample from the assignment. The optimisation module may be configured to request a new medical sample to replace said medical sample in the assignment.

In some examples the initial assignment may further comprises a priority ranking of the analytical tests, indicating the priority with which each test should be performed; and the optimisation module may modify the assignment based in part on the priority ranking.

The optimisation module may be configured to modify the assignment by being further configured to assign the medical sample to more than one analytical test. In such a case, the system may instruct the creation of an aliquot from said sample.

The optimisation module may be configured to provide the modified assignment to a laboratory scheduling system for automatic implementation of the modified assignment.

The optimisation module and compliance module as discussed herein may be implemented as elements of software or software suites running on one or more computers.

The system may be a laboratory information system. The system may be a healthcare information system. The system may be a hospital information system. The system may be a laboratory information management system.

The system may be a centralised system, in that each module may be located on a same computer or local computer-network or may be a de-centralised system in that one or more of the modules may be located in a different computer or local computer-network to one or more other modules.

The system may be implemented as laboratory middleware.

The system may be in networked communication with a sample transport system (e.g. a conveyor within a laboratory, or a vehicle transporting the or each medical sample) or a transport box containing the or each medical sample (for example a pre-pre-analytics transport box), such that the sample data can be obtained during transit of the sample.

In a third aspect there is provided a computer-implemented method for assessing the suitability of one or more of medical samples to one or more analytical tests, the method comprising the steps of: obtaining a sample quality metric for the or each medical sample; obtaining an analytical test specification for the or each analytical test; performing a compliance process of assessing, for the or each medical sample, whether its sample quality metric violates the analytical test specification of the or each analytical test; and for the or each medical sample, outputting an indication of whether its sample quality metric violates the analytical test specification of the or each analytical test.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
Figure 1 is a block diagram illustrating an analytical testing management system within a network;
Figure 2 is a flowchart for a method of optimising an assignment of one or more medical samples to one or more analytical tests to be conducted on those medical samples;
Figure 3 is a block diagram illustrating an analytical testing management system within a network;
Figure 4 is a flowchart for a method of obtaining a sample quality metric for a medical sample;
Figure 5 is a flowchart for a method of determining the compliance status of one or more medical samples for one or more analytical tests listed in an initial assignment of medical samples to analytical tests;
Figure 6 is a flowchart for a method of optimising an assignment of one or more medical samples to one or more analytical tests to be conducted on those medical samples that is a modification of the flowchart in Figure 2;
Figures 7A - 7C illustrate an initial assignment of medical samples to analytical tests, the results of determining the compliance status of those medical samples with the analytical tests, and a modified assignment of medical samples to analytical tests; and
Figure 8A - 8D illustrate an initial assignment of medical samples to analytical tests, the results of determining the compliance status of those medical samples with the analytical tests, and two possible modified assignment of medical samples to analytical tests.

### Detailed Description of the Invention

Aspects and embodiments of the present invention will now be discussed with reference to the accompanying figures. Further aspects and embodiments will be apparent to those skilled in the art.

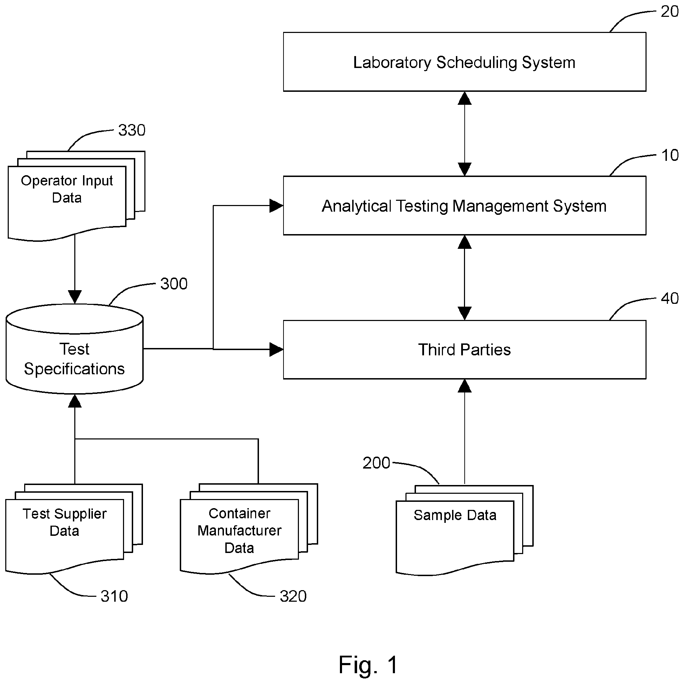

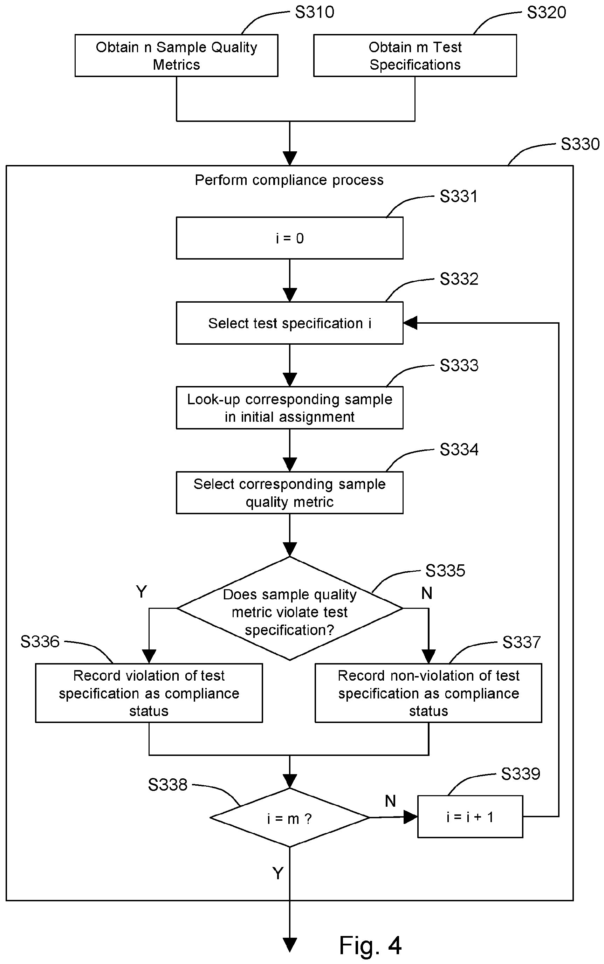

Figure 1 is a block diagram illustrating an analytical testing management system 10 within a network. The analytical testing management system 10 may be implemented as, or within, a laboratory information system, a healthcare information system or a laboratory information management system. The system 10 is configured within the network for optimising an assignment of one or more medical samples to one or more analytical tests to be conducted on those samples. The initial assignment of medical samples to analytical tests is provided to the system 10 from a laboratory scheduling system 20 that the system 10 is in networked communication with. The system 10 is also in networked communication with one or more third parties 40, which take the form of apps or services usable by the system 10 in order to obtain information for optimising the assignment of medical samples to analytical tests. The system 10 communicates with the third parties 40 to determine, for each medical sample, a compliance status indicating (i) whether a sample quality metric of the or each medical sample violates an analytical test specification of the or each of the medical sample's assigned analytical test(s) in the initial assignment and/or (ii) whether the or each medical sample is unprocessable. This determination is in part based on the sample data 200 that the third parties 40 collect about the medical samples. The determination is also based on analytical test specifications 300 for the tests in the initial assignment that are obtained by the system 10 and/or the third parties 40 (depending on how the determination of the compliance status is to be conducted, which is discussed further in relation to Figure 4 below). The test specifications 300 may be based on a number of sources of information, including data from the test supplier 310, data from the container manufacturer 320 whose containers are used during the tests, and data from the laboratory operator 330 that reflects, for example, experience gained in conducting previous tests. The analytical test specification 300 used in determining the compliance status of a medical sample in relation to an analytical test is looked up by the analytical testing management system 10 and/or the third parties 40 using a combination of a test code, for example, a LOINC code, and a description of the analytical test. Having obtained the compliance status of the medical samples for their respective analytical tests, the system 10 can perform an optimisation process prior to processing of said medical samples in an analytical laboratory to reduce the total number of test specification violations and/or the number of samples that are unprocessable. The optimised assignment of the medical samples to the analytical tests is output from the analytical testing management system 10 to the laboratory scheduling system 20. The optimisation of the assignment of the medical samples to the analytical tests may be conducted by a third party and subsequently the optimised assignment may be provided from the third party to the analytical testing management system 10 for distribution to the laboratory scheduling system 20; alternatively, the analytical testing management system 10 may itself conduct said optimisation.

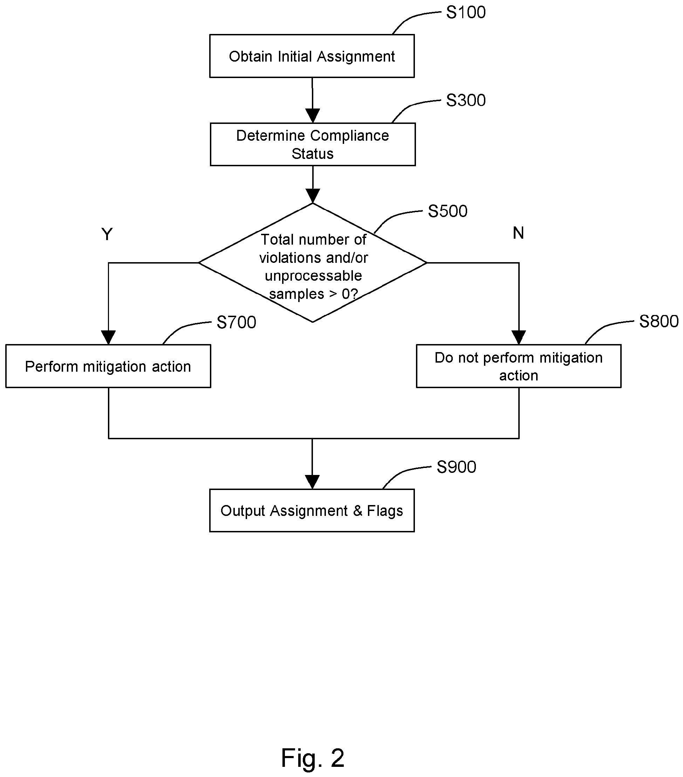

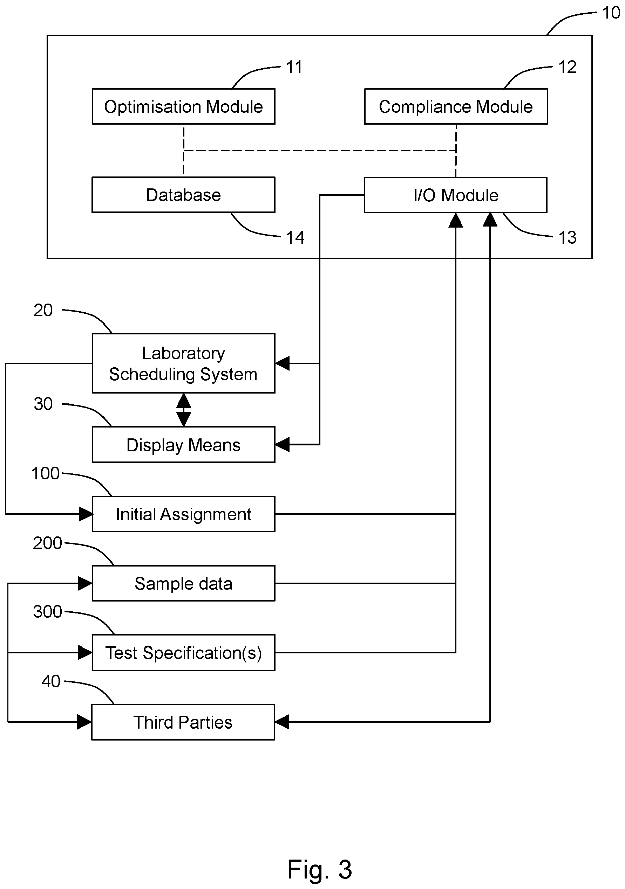

Figure 2 is a flowchart illustrating a computer-implemented method of optimising an assignment of one or more medical samples to one or more analytical tests to be conducted on those samples. The method illustrated by Figure 2 can be implemented by an analytical testing management system 10 such as that illustrated in Figure 3. In order for a given analytical test to be successfully conducted on a medical sample and produce a valid result, the medical sample should (i) have a sample quality metric that complies with an analytical test specification for the given analytical test and (ii) be processable.

Typically, a medical sample will be found to be unprocessable where one or more of the following events has occurred: the medical sample has not arrived at the intended destination (e.g. a laboratory) where the analytical test was to be conducted on it; the medical sample has been lost; the medical sample has been spilled, otherwise contaminated or tampered with; the medical sample was not collected; and an identification label for the sample (e.g. a barcode) has been lost or rendered unreadable.

On the other hand, an analytical test specification 300 specifies values of one or more metrics that must be met by the sample in order to conduct that analytical test on it. For example, a test specification 300 may relate to parameters such as: the time between obtaining a sample and conducting the test on the sample, minimum and maximum temperature exposure, centrifugation settings (where centrifugation is required), time between obtaining a sample and conducting centrifugation (where centrifugation is required), light exposure, maximum and minimum humidity exposure, shock exposure and sample container properties.

At step S100, an initial assignment 100 of medical samples to analytical tests is obtained. In Figure 3, this is obtained by the optimisation module 11 via the input/output (I/O) module 13. Typically, medical samples are collected with a particular analytical test in mind for each medical sample; the initial assignment 100 reflects the pairing of medical samples to analytical tests. A one-to-one relationship between samples and tests is common, however it is also possible for the initial assignment 100 to indicate that more than one analytical test is to be performed on one sample. Multiple analytical tests may be conducted on a sample by the creation of aliquots from the sample, or where the preceding analytical test(s) does not alter the sample.

At step S300, the compliance status of each of the medical samples with its corresponding analytical test(s) in the initial assignment 100 is determined. The analytical testing management system 10 comprises a compliance module 12 to execute step S300. In this example, a medical sample that is stated to be "compliant" with an analytical test may be understood to (i) be processable (i.e. is not unprocessable) and (ii) have one or more sample quality metrics that each comply with (i.e. do not violate) the analytical test specification 300. As discussed above however, in other examples only one of (i) and (ii) may be determined or the determination of (ii) may be dependent on a positive finding from the determination of (i). That is, the compliance module 12 may first determine that the medical sample is processable and only determine (ii) for each medical sample that is found to be processable. Where either (i) or (ii) is determined in the negative (the sample is unprocessable or violates analytical test specifications) the overall compliance status for that medical sample may be considered to be negative (i.e. it does not comply with at least one of (i) and (ii)).

One way to determine the compliance status of each medical sample in respect of an analytical test is to obtain, for each medical sample, from a remote device, an indication of whether the sample quality metric(s) of the sample violates the analytical test specification 300 of its assigned analytical test. For example, the remote device may be contained within the system that first obtained the medical sample and placed the analytical test order, and that system may have already considered the compliance status of the sample.

However, the system 10 in Figure 3 is configured to determine the compliance status of the medical samples in respect of their assigned analytical tests internally using the sample data 200 and test specifications 300 that it obtains via its I/O module 13. The steps involved in the system 10 of Figure 3 determining the compliance status is illustrated in Figure 4 and are discussed further below. The system 10 illustrated in Figure 3 obtains the test specifications 300 from an external source, i.e. a third party 40 such as a test supplier and/or a sample container manufacturer. This is typically done by providing the third party 40 with a LOINC code and/or another description of each analytical test that they can use to identify the appropriate test specifications 300 to provide back to the system 10. However, test specifications 300 may also be obtained from a database 14 internal to the system 10. The database 14 may store test specifications 300 that the system 10 has referred to previously, or may store test specifications 300 where these are modified from those provided by third parties 40 (for example, where feedback from the laboratory conducting the corresponding analytical tests has been incorporated into the test specifications 300).

The compliance status determined at step S300 may also indicate that a medical sample is unprocessable. In such an instance, the system 10 may be further configured to determine what the root cause of the medical sample being found to be unprocessable was (e.g. whether it was because the sample was lost, never arrived at the intended destination etc.). Whether or not a sample is unprocessable may be determined internally by the system (e.g. if sample data 200 for that sample is missing) or by a third party 40 that then informs the system 10 that the sample is unprocessable (e.g. if the third party 40 knows that the sample was lost in transit).

Having determined the compliance status of each of the medical samples in respect of their assigned analytical tests, Step S500 involves checking whether there is at least one compliance status that is negative, that is whether the total number of violations of test specifications 300 (i.e. the sum of the number of medical samples with a sample quality metric that violates the test specification 300 of the respective analytical tests assigned to them in the initial assignment 100) and/or the total number of unprocessable samples is greater than zero. If the check at step S500 is negative (there are no negative compliance statuses), the system 10 does not proceed to perform a mitigation action (step S800), since it has been established that the initial assignment of the medical samples to the analytical tests is appropriate and/or all medical samples are processable. However, if the check at step S500 is positive (there is at least one negative compliance status), then the system 10 proceeds to step S700 of the method, where mitigation action is taken by the optimisation module 11 in response to the determination that the initial assignment 100 of medical samples to analytical tests results in a violation of analytical test specification 300 (i.e. a test listed in the initial assignment 100 is likely to return an invalid result) and/or one or more of the samples listed in the initial assignment 100 is unprocessable (i.e. the corresponding test listed in the initial assignment 100 cannot be conducted on that sample).

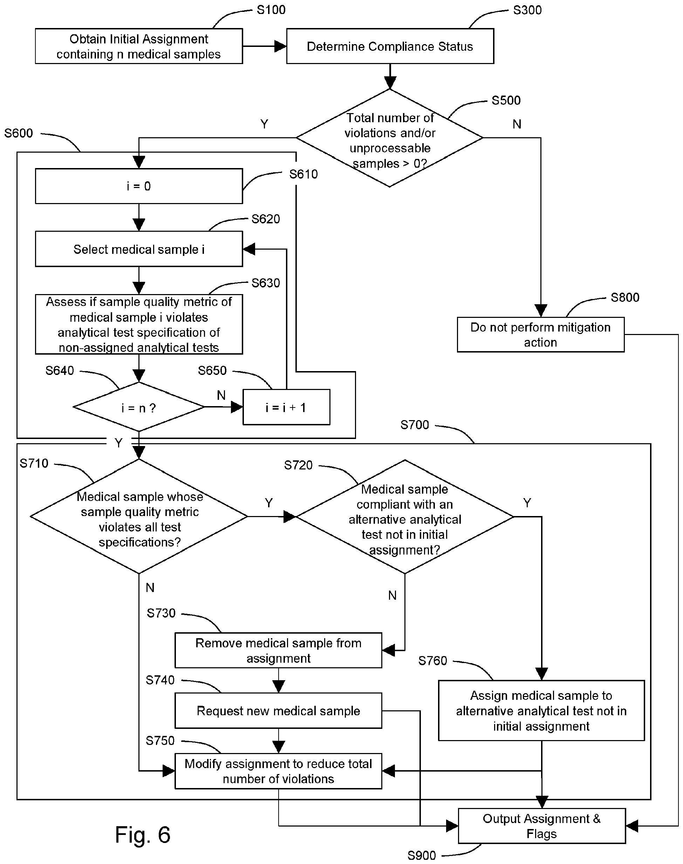

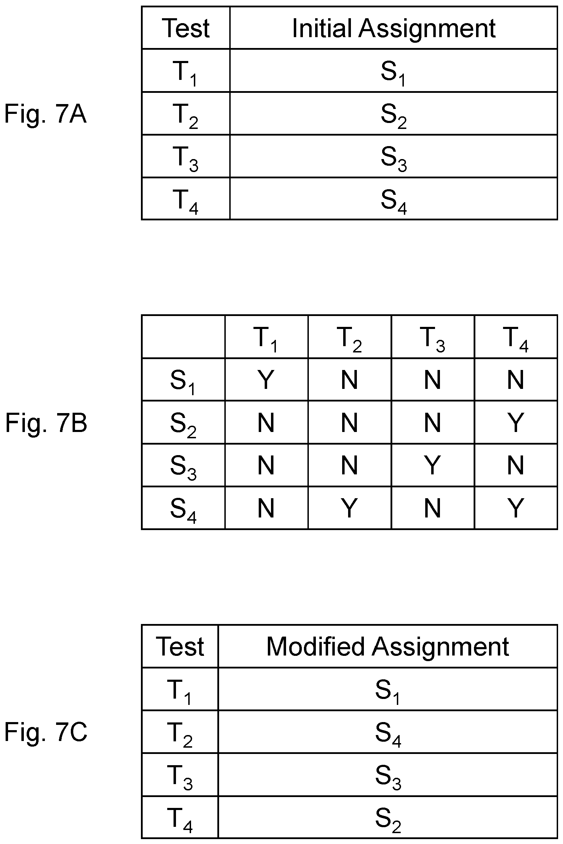

One possible type of mitigation action is for the optimisation module 11 to modify the assignment of the medical samples to the analytical tests based on the compliance status of the medical samples. This type of mitigation action is discussed further in relation to Figures 6-8 below. Where, for example, the assignment can be modified by swapping the assignment of two samples to two respective tests (see, for example, Figures 7A - 7C, where tests T2 and T4 have their assignments swapped between the initial assignment (Figure 7A) and the modified assignment (Figure 7C)), it is possible to reduce the number of medical samples that are discarded because their sample quality metrics are found not to comply with the analytical test specifications 300 of the tests they were initially assigned to and increase the proportion of the tests listed in the initial assignment 100 that can be conducted. The modification is typically conducted by performing a minimisation optimisation with the sum of test specification violations as an objective function.

Another possible type of mitigation action is for the optimisation module 11 to additionally flag and/or invalidate the test order(s) and/or the test result(s) associated with the medical samples that have a compliance status indicating that (i) a sample quality metric of the or each medical sample violates an analytical test specification 300 of the medical sample's assigned analytical test(s) in the modified assignment and/or (ii) the or each medical sample is unprocessable.

At step S900, the assignment and any flags raised are output from the system 10 via the I/O module 13. The flags, where present, may be notified to a laboratory operator via display means 30. The modified assignment, where present, may be notified to a laboratory operator for review via the display means 30. The notification to the operator may simply be for informational purposes, and the modified assignment may be automatically implemented by a laboratory scheduling system 20. Alternatively, the notification may be such that the operator can decide to accept or decline the proposed modified assignment.

Figure 4 illustrates a method by which the system 10 of Figure 3 determines the compliance status of the medical samples in respect of the assigned analytical tests in the initial assignment 100. In Figure 4, for simplicity, it is assumed that each medical sample has a single sample quality metric and each test specification specifies the value of that sample quality metric. However, as discussed above, it is possible for a medical sample to have plural sample quality metrics, all of which must be compliant with a test specification for the medical sample to be compliant with that analytical test.

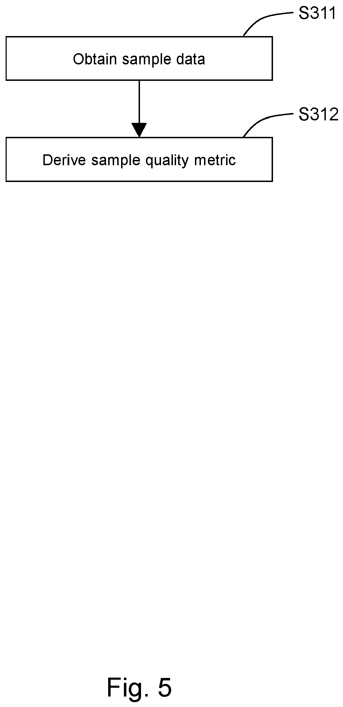

At step S310, n sample quality metrics are obtained by the compliance module 12, each sample quality metric corresponding to a respective medical sample listed in the initial assignment 100 obtained by the system 10 (i.e. n medical samples are listed in the initial assignment 100). The step of obtaining sample quality metrics is discussed further in relation to Figure 5. At step S320, m test specifications 300 are obtained by the compliance module 12, each test specification 300 corresponding to a respective analytical test listed in the initial assignment 100 obtained by the system 10. Steps S310 and S320 may be conducted in parallel with each other, or sequentially with each other in either order.

At step S330, a compliance process is performed by the compliance module 12 based on the sample quality metrics and test specifications 300 obtained at steps S310 and S320, respectively. In the case of the method illustrated in Figure 4, the compliances status determined is indicative of whether the sample quality metric of the medical samples violates the test specification 300 of its respective analytical test listed in the initial assignment 100.

Step S330 contains a plurality of sub-steps that represent an iterative process working through the m tests, for each one determining the compliance status of the respective medical sample. As step S331, a counter, i, for the iterative process is set to 0. At step S332, test specification i from the m test specifications 300 is selected. Step S333 is then a look-up using the initial assignment 100 to determine the sample corresponding to the test that test specification i relates to. Subsequently, at step S334, the sample quality metric corresponding to the sample looked up at step S333 is selected. At step S335, comparison of the sample quality metric to the test specification i is used to determine if the sample quality metric violates the test specification 300. Depending on the result of step S335, violation of the test specification 300 (step S336) or non-violation of the test specification 300 (step S337) is recorded as the compliance status of the medical sample in respect of analytical test i it is assigned to. Step 338 then checks whether all of the test specifications 300 have been considered; if they have not all been considered, the method returns to Step S332 via step S339 to select the next test specification 300; if they have all been considered, the compliance process step S330 is complete.

Figure 5 illustrates a method by which step S310 in Figure 4 of obtaining the sample quality metrics of the medical samples listed in the initial assignment 100 can be conducted. The method in Figure 5 is for obtaining the sample quality metric for a single medical sample. This method can be repeated until sample quality metrics for all the medical samples listed in the initial assignment 100 are obtained.

At step S311, sample data 200 is obtained. The sample data 200 is information about the medical sample that is obtained from data sources external to the system 10, so this sample data 200 is obtained by the compliance module 12 of the system 10 via the I/O module 13 that communicates with the external data sources. The sample data 200 may include one or more of: collection information for the sample, transport information from the sample, centrifugation information for the sample; and storage condition information of the sample. The collection information for a given sample may include one or more of: a collection time stamp, patient data, collection observations, images of the sample after collection, sample fixation time. Patient data may include one or more of: a patient ID, and patient demographics. The transport information for the sample may include one or more of: a sample temperature measurement during transport, a sample light exposure measurement during transport, a sample shock exposure measurement during transport and sample container additive information, and GPS data. The centrifugation information for the sample may include one or more of: an indication of whether centrifugation has been conducted, a collection-to-centrifugation time measurement and a centrifugation specification. The storage condition information for the sample may include one or more of: a temperature measurement during storage, a sample light exposure measurement during storage, a humidity measurement during storage, a sample shock exposure measurement during storage, and a storage duration measurement.

At step S312, the sample quality metric for a medical sample is derived based on the sample data 200 for that sample. Where reference is made to a sample quality metric, said metric may be a parameter or a statistic. It is also the case that where reference is made to a singular sample quality metric, it is possible for plural sample quality metrics to be present, the sample quality metrics optionally being a combination of one or more parameters and/or one or more statistics. A common sample quality metric is the heamoglobin (H), icterus (I), and lipaemia (L) indices in a serum or plasma (H/I/L indices).

Figure 6 is a flowchart for a method of optimising an assignment of one or more medical samples to one or more analytical tests to be conducted on those medical samples. The flowchart in Figure 6 is a modification of the flowchart in Figure 2. Figure 6 includes an additional step S600 of determining, for each of the samples, whether its sample quality metric violates the analytical test specification 300 of each of the analytical tests in the initial assignment 100 other than its assigned analytical test(s) in the initial assignment 100. Figure 6 also includes additional detail for step S700 on the sub-steps involved in performing one example of a mitigation action.

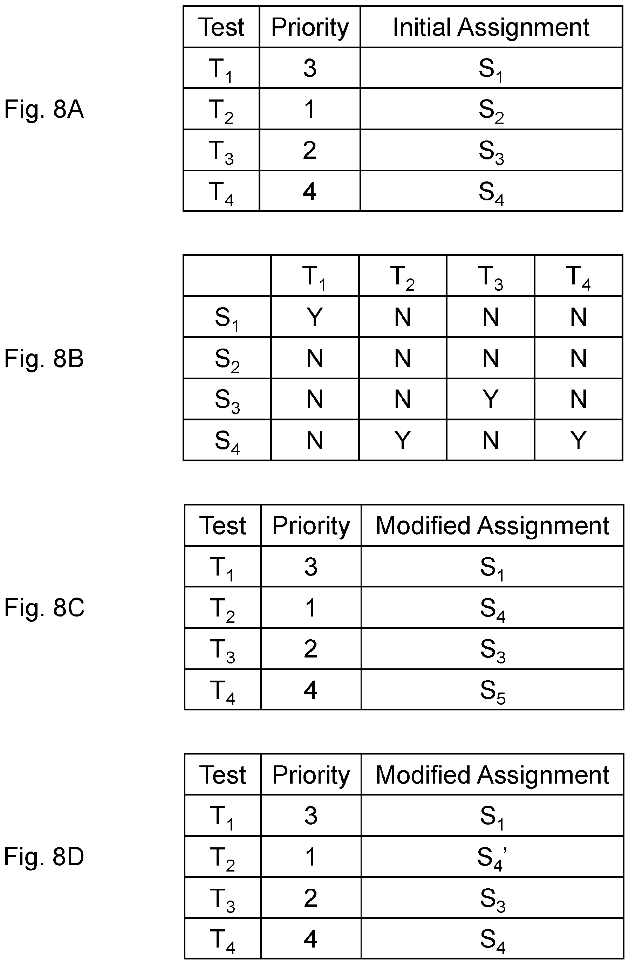

The method illustrated by the flowchart of Figure 6 is described below in conjunction with Figures 7A - 7C and 8A - 8D. Figure 7A is a table that illustrates the initial assignment 100 of four medical samples (S₁ - S₄) to four analytical tests (T₁ - T₄). Figure 7B is a table that illustrates the compliance status (Y or N, corresponding to compliant and non-compliant, respectively) of each of the sample quality metrics of medical samples S₁ - S₄ in respect of each of the test specifications 300 of analytical tests T₁ - T₄ in Figure 7A. Figure 7C is a table that illustrates a modified assignment of the medical samples S₁ - S₄ to the analytical tests T₁ - T₄. Similar to Figure 7A, Figure 8A is a table that illustrates the initial assignment 100 of four medical samples S₁ - S₄ to four analytical tests T₁ - T₄; however, Figure 8A also includes a priority ranking of the analytical tests, indicating the priority with which each test should be performed, with test T₂ ranked the highest (most important to perform) and test T₄ ranked the lowest (least important to perform). Similar to Figure 7B, Figure 8B is a table that illustrates the compliance status (Y or N, corresponding to compliant and non-compliant, respectively) of each of the sample quality metrics of medical samples S₁ - S₄ in respect of each of the test specifications 300 of analytical tests T₁ - T₄ in Figure 8A. Figures 8C and 8D are tables with alternative modified assignments of medical samples to the analytical tests T₁ - T₄.

Steps S100, S300, S500, S800 and S900 in Figure 6 are the same as steps S100, S300, S500, S800 and S900 described above in relation to Figure 2 and accordingly a description of these steps is omitted from the description of Figure 6.

The flowchart of Figure 6 differs from that of Figure 2 in that, if the check at step S500 is positive, the flowchart in Figure 6 proceeds to Step S600, where it is determined, for each of the medical samples in the initial assignment 100, whether its sample quality metric violates the analytical test specification 300 of each of the analytical tests in the initial assignment 100 other than its assigned analytical test(s) in the initial assignment 100. Step S600 is conducted by the compliance module 12 within the system 10 of Figure 3. In Figure 6 step S600 is shown to comprise a plurality of sub-steps S610 - S650 that constitute an iterative loop for assessing each medical sample. At step S610, a counter, i, for the iterative process is set to 0. At step S620, medical sample i from the n medical samples is selected. Step S630 then involves assessing if the sample quality metric of medical sample i violates the analytical test specifications 300 of the tests it was not assigned to in the initial assignment 100, the result of which is recorded. Step S640 then checks whether all of the medical samples have been considered; if they have not all been considered, the method returns to step S620 via step S650 to select the next medical sample; if they have all been considered, then step S600 is complete.

Turning to Figures 7A and 7B, in the case of the initial assignment 100 in Figure 7A, the check at step S500 is positive since, as Figures 7A and 7B show, sample S₂ is initially assigned to test T₂, but the sample quality statistic of S₂ violates the test specification 300 of T₂, meaning the method progresses to step S600. Step S600 in Figure 6 can be understood as determining whether sample S₁'s sample quality metric violates the test specifications 300 of analytical tests T₂ - T₄ (since S₁ is assigned to T₁ in Figure 7A), whether sample S₂'s sample quality metric violates the test specifications 300 of analytical tests T₁, T₃ and T₄ (since S₂ is assigned to T₂ in Figure 7A), whether sample S₃'s sample quality metric violates the test specifications 300 of analytical tests T₁, T₂ and T₄ (since S₃ is assigned to T₃ in Figure 7A), and whether sample Sa's sample quality metric violates the test specifications 300 of analytical tests T₁ - T₃ (since S₄ is assigned to T₄ in Figure 7A). The results of step S600 are shown in Figure 7B: sample S₂ is compliant with test T₄ and sample S₄ is compliant with test T₂, but otherwise the medical samples are not compliant with tests that they were not assigned to in the initial assignment 100.

Following step S600, the flowchart in Figure 6 proceeds to step S700 where mitigation action is taken by the optimisation module 11 of the system 10 in Figure 3. The mitigation action represented by step S700 of Figure 6 is an example of the mitigation action that may be taken in response to a positive determination at step S500. Step S700 in Figure 6 comprises a plurality of sub-steps S710 - S760.

Firstly, at Step 710 it is checked whether there is a medical sample in the initial assignment 100 whose sample quality metric violates all of the test specifications 300 of all of the analytical tests in the initial assignment 100. By way of example, the table in Figure 7B does not contain any samples that violate all of the test specifications 300 in the initial assignment, as each of samples S₁ - S₄ are compliant with at least one test of tests T₁ - T₄ (even if that test is not the test the sample is assigned to in the initial assignment 100). By contrast, in Figure 8B, sample S2 is shown to violate the test specifications 300 of all of tests T₁ - T₄ listed in the initial assignment 100. Accordingly, the check at step S710 would be answered negatively for the samples and tests in Figure 7B, and positively for the samples and tests in Figure 8B.

Considering first the example of the samples and tests in Figure 7B, the method progresses to step S750, where the assignment of medical samples to analytical tests is modified in order to reduce the total number of violations of test specifications 300 that occur in comparison to the initial assignment 100. The process of modifying the initial assignment 100 may further include identifying a medical sample whose sample quality statistic violates the analytical test specification 300 of the analytical test(s) it is assigned to in the initial assignment 100 - sample S₂ has a sample quality statistic that violates the analytical test specification of test T₂. Step S750 may further include identifying, for medical sample S₂, whether there is an alternative analytical test other than its assigned analytical test in the initial assignment 100 that its sample quality metric does not violate the analytical test specification 300 of; in the case of Figure 7B, S₂'s sample quality statistic does not violate the test specification 300 of test T₄. Test T₄ is assigned medical sample S₄ in the initial assignment 100 (see Figure 7A), and sample S₄ is compliant with the test specifications 300 of tests T₂ and T₄ (see Figure 7B). Accordingly it is possible for the assignment to be modified such that sample S₂, which is not compliant with its initially assigned test, to be re-assigned to alternative analytical test T₄ that it is compliant with, and for sample S₄, which has been replaced by S₂ as the sample assigned to T₄, to be re-assigned to T₂, whose test specification 300 it complies with. The modified assignment is shown in the table of Figure 7C. Accordingly, all of the analytical tests listed in the initial assignment 100 can be performed on medical samples whose sample quality statistics meet the respective test specifications 300 and all of the medical samples listed in the initial assignment 100 are used in conducting those analytical tests.

In the example illustrated by Figures 7A - 7C, the alternative analytical test T₄ that sample S₂ is re-assigned to is an analytical test that is present in the initial assignment 100.

Considering the example of the samples and tests in Figure 8B, the method progresses to step S720 from step S710 in Figure 6 because sample S₂ violates the analytical test specifications of all of tests T₁ - T₄ in the initial assignment 100. At step S720 it is checked whether sample S₂ is compliant with an alternative analytical test that is not present in the initial assignment 100. For example, it is checked whether there is an alternative test that is conducted for the same purpose (and possibly even to obtain the same parameter) as the initially assigned test T₂ but whose test specification sample S₂'s sample quality metric complies with. If it is possible to identify such an alternative analytical test, the method progresses to step S760 where sample S₂ is assigned to the alternative analytical test that is not present in the initial assignment 100. Following the assignment of the medical sample to the alternative analytical test not in the initial assignment 100, the method proceeds to step S750, where it is further attempted to modify the assignment of tests to samples in order to try to further reduce the total number of violations. However, it is also possible that the total number of violations is now zero following step S740, in which case the assignment is not further modified at step S750.

However, in the case of Figures 8A - 8D, no such alternative analytical test is identified, and accordingly the method progresses to step S730 in Figure 6. At step S730, the medical sample S₂ is removed from the assignment of samples to tests because it's sample quality metric violates all of the test specifications 300 of the tests in the initial assignment 100 and any alternative analytical tests not in the initial assignment and is therefore not of use. Subsequently, at step S740, a new medical sample is requested to replace sample S₂. The requesting of a new medical sample may be executed in a number of different manners. One option illustrated in Figure 8C is for a completely new sample to be requested, in this case sample S₅. The compliance status of the newly requested sample S₅ may also be determined in respect of the analytical test it is to be assigned to. An alternative option illustrated in Figure 8D is for one medical sample to be assigned to more than one analytical test. Where an analytical test within the plural analytical tests alters the sample, the assignment of the one medical sample to the plural analytical tests may involve instructing the creation of an aliquot from the medical sample assigned to plural analytical tests. However, where the plural analytical tests do not alter the sample (for example, a spectroscopic analytical test), the assignment of a medical sample to plural analytical tests may not require the creation of an aliquot. In Figure 8D, sample S₄ is assigned to test T₄ (which it was initially assigned to and is compliant with), and an aliquot of sample S₄ (indicated by S₄' in Figure 8D) is assigned to test T₂ in order to replace sample S₂, since the sample quality metric of sample S₄ is also compliant with the analytical test specification 300 of test T₂ (see Figure 8B).

Additionally, as mentioned above, in Figures 8A, 8C and 8D, a priority ranking of the analytical tests is provided, indicating the priority with which each test should be performed. The step of modifying the assignment to provide the modified assignment in Figure 8C was based on the priority ranking. This can be seen by the assignment of Figure 8C, where, rather than a new sample being requested for test T₂ -which is the highest priority test -, the sample S₄ has been re-assigned to test T₂ and the requested new sample S₅ is assigned to test T₄, which has a lower priority ranking than test T₂. In this manner, any time lag associated with requesting and obtaining a new sample delays the ability to execute test T₄ rather than test T₂, which is preferable where test T₂ has a higher priority. The priority raking may be a weighted sum, e.g. a weighted sum based on factors including: how suitable a given sample is for a given test; whether the test is given an `URGENT' status or not; what fraction of a sample's volume is used by a given test; the time-sensitivity of obtaining a test result; and whether the test result is essential or supporting, for example, for a diagnosis. Typically, the priority ranking is based on a test order placer preference and/or a lab preference.

Following the requesting of a new medical sample, the method proceeds to step S750, where it is further attempted to modify the assignment of tests to samples in order to try to further reduce the total number of violations. However, it is also possible that the total number of violations is now zero following step S740, in which case the assignment is not further modified at step S750.

The term 'laboratory instrument' as used herein encompasses any apparatus or apparatus component operable to execute one or more processing steps / workflow steps on one or more biological samples and/or one or more reagents. The term 'instrument' covers pre-analytical instruments, post-analytical instruments and also analytical instruments.

The term 'analyzer' / 'analytical instrument' as used herein encompasses any apparatus or apparatus component configured to obtain a measurement value. An analyzer is operable to determine via various chemical, biological, physical, optical or other technical procedures a parameter value of the sample or a component thereof. An analyzer may be operable to measure said parameter of the sample or of at least one analyte and return the obtained measurement value. The list of possible analysis results returned by the analyzer comprises, without limitation, concentrations of the analyte in the sample, a digital (yes or no) result indicating the existence of the analyte in the sample (corresponding to a concentration above the detection level), optical parameters, DNA or RNA sequences, data obtained from mass spectrometry of proteins or metabolites and physical or chemical parameters of various types. An analytical instrument may comprise units assisting with the pipetting, dosing, and mixing of samples and/or reagents. The analyzer may comprise a reagent holding unit for holding reagents to perform the assays. Reagents may be arranged for example in the form of containers or cassettes containing individual reagents or group of reagents, placed in appropriate receptacles or positions within a storage compartment or conveyor. It may comprise a consumable feeding unit. The analyzer may comprise a process and detection system whose workflow is optimized for certain types of analysis. Examples of such analyzers are clinical chemistry analyzers, coagulation chemistry analyzers, immunochemistry analyzers, urine analyzers, nucleic acid analyzers, used to detect the result of chemical or biological reactions or to monitor the progress of chemical or biological reactions.

The term 'pre-analytical instrument' as used herein encompasses any apparatus or apparatus component that is configured to perform one or more pre-analytical workflow steps comprising - but not limited to - centrifugation, resuspension (e.g. by mixing or vortexing), capping, decapping, recapping, sorting, tube type identification, sample quality determination and/or aliquotation steps. Said steps may also comprise adding chemicals or buffers to a sample, concentrating a sample, incubating a sample, and the like.

The term 'post-analytical instrument' as used herein encompasses any apparatus or apparatus component that is configured to perform one or more post-analytical workflow steps comprising - but not limited to - sample unloading, transport, recapping, decapping, temporary storage / buffering, archiving (refrigerated or not), retrieval and/ or disposal.

The term 'point-of-care device' as used herein encompasses any analyzer used in a point-of-care environment, such as (but not limited to) blood glucose testing, coagulation testing, blood gas and electrolytes analysis, urinalysis, cardiac markers analysis, hemoglobin diagnostics, infectious disease testing, cholesterol screening or nucleic acid testing NAT. Results may be viewed directly on the POC analyzer/device(s) or may be sent to and displayed in a healthcare information management system.

The term 'connected' as used herein encompasses both direct and indirect communication pathways between two or more elements. A communications pathway may be provided through a physical entity such as a wired connection or may be provided through a non-physical communication system such as a network.

The systems and methods of the above embodiments may be implemented in a computer system (in particular in computer hardware or in computer software) in addition to the structural components and user interactions described.

The term 'computer system' includes the hardware, software and data storage devices for embodying a system or carrying out a method according to the above described embodiments. For example, a computer system may comprise a central processing unit (CPU), input means, output means and data storage. The computer system may have a monitor to provide a visual output display. The data storage may comprise RAM, disk drives or other computer readable media. The computer system may include a plurality of computing devices connected by a network and able to communicate with each other over that network, and in such instances may be referred to as a computer network.

The methods of the above embodiments may be provided as computer programs or as computer program products or computer readable media carrying a computer program which is arranged, when run on a computer, to perform the method(s) described above.

The term 'computer readable media' includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Reference Signs

| | |
|---|---|
| Analytical testing management system | 10 |
| Optimisation module | 11 |
| Compliance module | 12 |
| I/O module | 13 |
| Database | 14 |
| Laboratory scheduling system | 20 |
| Display means | 30 |
| Third party | 40 |
| Initial assignment | 100 |
| Sample data | 200 |
| Test specifications | 300 |
| Test supplier data | 310 |
| Container manufacturer data | 320 |
| Operator input data | 330 |

## Claims

1. A computer-implemented method for optimising an assignment of one or more medical samples to one or more analytical tests to be conducted on those medical samples prior to processing of said medical samples in an analytical laboratory, the method comprising the steps of:
obtaining an initial assignment (100) of the one or more medical samples to the one or more analytical tests;
determining a compliance status for the or each medical sample, the compliance status indicating: (i) whether a sample quality metric of the or each medical sample violates an analytical test specification (300) of the medical sample's assigned analytical test(s) in the initial assignment (100) and/or (ii) whether the or each medical sample is unprocessable; and
performing, prior to processing of said medical sample, a mitigation action if one or more compliance status indicate: (i) that the sample quality metric of a given medical sample violates the analytical test specification (300) and/or (ii) a given medical sample is unprocessable, wherein the mitigation action includes a step of modifying the assignment of the medical sample(s) to the analytical test(s) based on the or each compliance status.

2. The computer implemented method of claim 1, further comprising, following the modification of the assignment, forwarding instructions to a laboratory scheduling system to process the medical sample according to the modified assignment to generate a test result.

3. The computer-implemented method of claim 1 or 2, wherein the mitigation action further includes a step of flagging and/or invalidating a test order associated with the analytical test(s) based on the or each compliance status of the analytical test for the sample according to the modified assignment.

4. The computer-implemented method of any of claims 1 to 3, wherein the method further comprises a step of deriving the sample quality metric for the or each medical sample, said step including:
obtaining sample data (200) from a remote agent including one or more of: collection information for the sample, transport information for the sample, centrifugation information for the sample; and storage condition information for the sample; and
deriving the sample quality metric based on the sample data (200).

5. The computer-implemented method of any preceding claim, wherein determining the compliance status for the or each medical sample includes either:
obtaining, for the or each medical sample, from a remote device, an indication of whether the sample quality metric of the medical sample violates the analytical test specification (300) of its assigned analytical test(s) in the initial assignment (100);
or:
obtaining the sample quality metric for the or each medical sample;
obtaining the analytical test specification for the or each analytical test in the initial assignment (100);
and
performing a compliance process of assessing, for the or each medical sample, whether the sample quality metric violates the analytical test specification (300) of its assigned analytical test(s) in the initial assignment (100).

6. The computer-implemented method according to any of the preceding claims, wherein the step of modifying the assignment further comprises:
identifying a medical sample whose sample quality metric violates the analytical test specification (300) of its assigned analytical test(s) in the initial assignment (100);
identifying, for that medical sample, an alternative analytical test other than its assigned analytical test(s) in the initial assignment (100) that its sample quality metric does not violate the analytical test specification (300) of; and
modifying the assignment such that said medical sample is assigned to the alternative analytical test.

7. The computer-implemented method according to claim 6, wherein the alternative analytical test is a test present in the initial assignment (100).

8. The computer-implemented method according to claim 6, wherein the alternative analytical test is a test that is not present in the initial assignment (100).

9. The computer-implemented method according to any preceding claim, further comprising a step of assessing, for the or each medical sample, whether its sample quality metric violates the analytical test specification (300) of the or each of the analytical test(s) in the initial assignment (100) other than its assigned analytical test(s) in the initial assignment (100).

10. The computer-implemented method according to claim 9, wherein the step of modifying the assignment further comprises:
identifying a medical sample whose sample quality metric violates all of the analytical test specifications (300) of the analytical tests in the initial assignment (100);
determining, for said medical sample, that there is not an alternative analytical test that is not present in the initial assignment (100) that its sample quality metric does not violate the analytical test specification (300) of; and
removing said medical sample from the assignment.

11. The computer-implemented method according to claim 10, further comprising a step of requesting a new medical sample to replace said medical sample in the assignment.

12. The computer-implemented method according to any of the preceding claims, wherein:
the initial assignment (100) further comprises a priority ranking of the analytical tests, indicating the priority with which each test should be performed; and
the step of modifying the assignment is based in part on the priority ranking.

13. The computer-implemented method according to any of the preceding claims, wherein:
the step of modifying the assignment further comprises assigning a medical sample to more than one analytical test.

14. The computer-implemented method according to any of the preceding claims, wherein said method is performed by an analytical testing management system which is communicatively connected to a Laboratory Scheduling System (20) and wherein said analytical testing management system obtains said initial assignment from said Laboratory Scheduling System (20) before processing of said medical sample according to said initial assignment.

15. An analytical testing management system (10) for optimising an assignment of one or more medical samples to one or more analytical tests to be conducted on those medical samples, the system comprising:
an optimisation module (11) configured to obtain an initial assignment (100) of the one or more medical samples to the one or more analytical tests prior to processing of said medical samples in an analytical laboratory; and
a compliance module (12) configured to:
determine, for the or each medical sample, a compliance status indicating: (i) whether a sample quality metric of the or each medical sample violates an analytical test specification (300) of the medical sample's assigned analytical test(s) in the initial assignment (100) and/or (ii) whether the or each medical sample is unprocessable; and
wherein the optimisation module (11) is further configured to perform a mitigation action prior to processing of said medical samples in an analytical laboratory if one or more compliance status indicates: (i) that the sample quality metric of a given medical sample violates the analytical test specification (300) and/or (ii) whether a given medical sample is unprocessable, wherein the mitigation action includes a step of modifying the assignment of the medical sample(s) to the analytical test(s) based on the or each compliance status.
